# EUROPEAN PATENT APPLICATION

(11) **EP 2 505 170 A1**
(43) Date of publication of application: **03.10.2012**
(21) Application number: 10833000.2
(22) Date of filing: 19.10.2010
(51) Int. Cl.: A61F 13/15, A61F 13/472, A61F 13/49

(54) **DEVICE AND METHOD FOR MANUFACTURING ABSORPTION BODY**

(30) Priority: 27.11.2009 JP 2009270635
(71) Applicant: Uni-Charm Corporation, Ehime 799-0111 (JP)
(72) Inventor: HOSHIKA, Kazuhiko, Kanonji-shi Kagawa 769-1602 (JP); IMAI, Atsushi, Kanonji-shi Kagawa 769-1602 (JP); ISSHIKI, Hiroshi, Kanonji-shi Kagawa 769-1602 (JP)
(74) Representative: Peter, Julian
(86) International application number: PCT/JP2010/068312
(87) International publication number: WO 2011/065153

(57) **Abstract**

A manufacturing device for an absorption body including an accumulation portion that is to have accumulated thereto liquid absorbent fiber in a first gas flowing through a duct and a polymer discharge tube that is introduced into the duct and that discharges from a discharge hole a second gas having high-absorbent polymer mixed therein toward the accumulation portion, wherein a flow path of the second gas formed in the polymer discharge tube has at a location on the discharge hole side of the flow path a distribution change area where a distribution state of high-absorbent polymer is changed, and a sectional area of the flow path at the distribution change area is narrower than a sectional area of a flow path at a part adjacent on an upstream side as well as a downstream side of the distribution change area.

## Description

### [Technical Field]

The present invention relates to a manufacturing device for absorbent bodies relating to absorbent articles such as disposable diapers and the like and a manufacturing method therefor.

### [Background Art]

Disposable diapers and sanitary napkins are conventionally known as absorbent articles that absorb fluid such as excreted fluids and the like. This absorbent article includes as a component thereof an absorbent body that absorbs fluid, and has particulate high-absorbent polymer (which is high molecular weight polymer and the like having high fluid retaining performance by swelling and the like due to fluid absorption, and is referred to as SAP hereunder) mixed therein and is produced by forming liquid absorbent fiber such as pulp fibers into a predetermined shape. Note that the absorbent body has a longitudinal direction and a width direction orthogonal to each other and a thickness direction as well. Among these, the width direction is oriented in the width direction of disposable diapers and sanitary napkins.

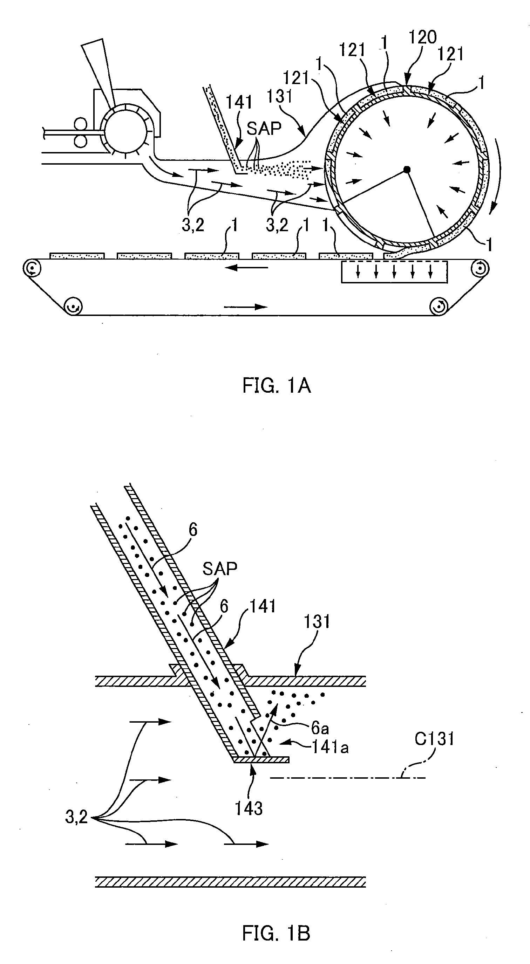

Such absorbent body 1 is for example, formed by accumulating pulp fiber 2 in the airflow 3 flowing through the duct 131 onto the accumulation portion 121 on the outer circumferential surface of the rotating drum 120, as shown in a schematic drawing of Fig. 1A. In other words, the accumulation portion 121 has multiple air intake holes (not shown) arranged in a predetermined arrangement and the air taken in therethrough makes the pulp fiber 2 accumulate to have a profile approximately the same as that of the accumulation portion 121 to create the absorbent body 1.

Additionally, the duct 131 has a polymer discharge tube 141 disposed thereto and SAP is discharged from the discharge hole 141a into the duct 131. In this way, SAP also flows along with the airflow 3 in the duct 131 to accumulate on the accumulation portion 121 with pulp fiber 2.

Here, the SAP and pulp fiber 2 are required to accumulate in a predetermined distribution state in the absorbent body 1. And SAP, in particular, generally needs to be evenly distributed along the width direction of the absorbent body 1.

With regard to this point, PTL1 discloses, as a method of distributing SAP evenly, a method of discharging SAP in a distributed manner into the duct 131 by placing a distribution plate 143 on the outer side of the discharge hole 141a of the polymer discharge tube 141 and making SAP flowing along the airflow 6 in the above discharge tube 141 to collide with the distribution plate 143.

### [Citation List]

### [Patent Literature]

[PTL 1] Japanese Patent Application Laid-open Publication No. 2009-112347

### [Summary of Invention]

### [Technical Problem]

However, the airflow 3 for allowing the pulp fiber 2 in the duct 131 to flow would be disturbed by the energetic airflow 6 distributed after colliding with the distribution plate 143. For example, in the example shown in Fig. 1B, a flow 6a in a direction approximately perpendicular to the duct axis C131 of the duct 131 will be generated and thereby disturbing the airflow 3 along the duct axis C131 of the duct 131. That is, there is fear that the airflow 3 along which pulp fiber 2 flows will be affected by the flow 6a changed for distributing SAP and as a result, the pulp fiber 2 would be unevenly distributed.

The present invention has been made in view of the conventional problems such as those mentioned above, and an object thereof is to provide a manufacturing device and a manufacturing method of an absorbent body capable of changing the high-absorbent polymer distribution state while restricting as much as possible effects on the flow of liquid absorbent fiber such as pulp fiber in a duct.

### [Solution to Problem]

In order to solve the above-described problem, a principal aspect of the invention is, a manufacturing device for an absorption body including an accumulation portion that is to have accumulated thereto liquid absorbent fiber in a first gas flowing through a duct, and a polymer discharge tube that is introduced into the duct and that discharges from a discharge hole a second gas having high-absorbent polymer mixed therein toward the accumulation portion, wherein a flow path of the second gas formed in the polymer discharge tube has at a location on the discharge hole side of the flow path a distribution change area where a distribution state of high-absorbent polymer is changed, and a sectional area of the flow path at the distribution change area is narrower than a sectional area of a flow path at a part adjacent on an upstream side as well as a downstream side of the distribution change area.

And a further aspect of the invention is, a method of manufacturing an absorbent body including accumulating liquid absorbent fiber in a first gas flowing through a duct to an accumulation portion, and discharging from a discharge hole of a polymer discharge tube being introduced into the duct a second gas having high-absorbent polymer mixed therein toward the accumulation portion, wherein a flow path of the second gas formed in the polymer discharge tube has at a location on the discharge hole side of the flow path a distribution change area where a distribution state of high-absorbent polymer is changed, and a sectional area of the flow path at the distribution change area is narrower than a sectional area of a flow path at a part adjacent on an upstream side as well as a downstream side of the distribution change area.

Features of the invention other than the above will become clear from the description of the present specification and the drawings attached.

### [Advantageous Effects of Invention]

According to the present invention, it is possible to change the distribution state of high-absorbent polymer while restricting as much as possible effects on the flow of liquid absorbent fiber such as pulp fiber in the duct.

### [Brief Description of Drawings]

[Fig. 1A] Fig. 1A is a schematic diagram of a conventional manufacturing device for absorbent bodies 1.
[Fig. 1B] Fig. 1B is an enlarged view of a portion proximate the discharge hole 141a of the polymer discharge tube 141.
[Fig. 2] Fig. 2 is a schematic diagram of a manufacturing device 10 for absorbent bodies 1 according to the present embodiment showing a vertical sectional view of the manufacturing device 10 cut along the central portion thereof.
[Fig. 3A] Fig. 3A is a vertical sectional view of the polymer discharge tube 41 cut along the central portion thereof.
[Fig. 3B] Fig. 3B is an enlarged view of part B in Fig. 3A.
[Fig. 3C] Fig. 3C is a sectional view taken along line C-C of Fig. 3B.
[Fig. 4A] Fig. 4A is an explanatory diagram showing the state of SAP being evenly distributed within the section of polymer discharge tube 41.
[Fig. 4B] Fig. 4B is a graph showing the amount of SAP distributed in the CD direction in an evenly distributed state.
[Fig. 5A] Fig. 5A is a vertical sectional view of another example of the distribution change area 45 cut along the central portion thereof.
[Fig. 5B] Fig. 5B is a sectional view taken along line B-B of Fig. 5A.
[Fig. 6A] Fig. 6A is a vertical sectional view of another example of the distribution change area 45.
[Fig. 6B] Fig. 6B is a vertical sectional view of further another example of the distribution change area 45.
[Fig. 7] Fig. 7 is a perspective view of a portion proximate the discharge hole 41a of the polymer discharge tube 41.
[Fig. 8A] Fig. 8A is a vertical sectional view of another example of the distribution change area 45.
[Fig. 8B] Fig. 8B is a sectional view taken along line B-B of Fig. 8A.
[Fig. 9A] Fig. 9A is a vertical sectional view of another example of the distribution change area 45.
[Fig. 9B] Fig. 9B is a sectional view taken along line B-B of Fig. 9A.

### [Mode for Carrying Out the Invention]

At least the following matters will be made clear from the description of the present specification with reference to the accompanying drawings.

A manufacturing device for an absorption body according to the present invention includes an accumulation portion that is to have accumulated thereto liquid absorbent fiber in a first gas flowing through a duct, and a polymer discharge tube that is introduced into the duct and that discharges from a discharge hole a second gas having high-absorbent polymer mixed therein toward the accumulation portion, wherein a flow path of the second gas formed in the polymer discharge tube has at a location on the discharge hole side of the flow path a distribution change area where a distribution state of high-absorbent polymer is changed, and a sectional area of the flow path at the distribution change area is narrower than a sectional area of a flow path at a part adjacent on an upstream side as well as a downstream side of the distribution change area.

According to such a manufacturing device for absorbent bodies, since the sectional area of the flow path at the distribution change area is small, the distribution of SAP is changed when SAP flows through the corresponding distribution change area and therewith the SAP distribution state in the duct after being discharged from the polymer discharge tube is also changed. As a result, the distribution of SAP accumulated in the absorbent body can be changed.

Further, the distribution change area is provided in the polymer discharge tube. Therefore, the effects from the changes in the second gas flow when changing the SAP distribution state can be effectively restrained from affecting the first gas flow on the outer side of the polymer discharge tube. In other words, the SAP distribution state can be changed without a significant effect on the flow of liquid absorbent fiber in the duct.

It is preferable that in the manufacturing device for an absorption body, a section relating to a sectional area of the flow path is an imaginary plane having a direction of flow through the flow path in a normal direction thereof, a shape of the section, when two directions orthogonal to each other and included in the imaginary plane are set as a first direction and a second direction, respectively, is a shape line-symmetric against a straight line parallel to the first direction running through a center of the sectional area, and a sectional shape of a flow path at the distribution change area being such that a clearance in the first direction at the central portion in the second direction is of a constricted shape narrower than parts on two sides of the distribution change area.

According to such a manufacturing device for absorbent bodies, the SAP distribution state can be changed so that the amount of SAP is reduced at the central portion in the second direction and the amount of SAP is increased on both end sides of the same second direction. In this way, it is possible to handle a phenomenon where the amount of SAP is increased in the central portion of the absorbent body caused by individual circumstances such as the sectional shape of the polymer discharge tube, or cases such as where there is a desire to increase the amount of SAP at the above-mentioned portions on both sides due to the design specification of the absorbent body. By the way, the former phenomenon where the amount of SAP is increased at the central portion of the absorbent body is likely to occur when using as the polymer discharge tube, for example, a tubular material whose flow path has a circular section.

It is preferable that in the manufacturing device for an absorption body, a sectional shape of a flow path of a part on the discharge hole side than the distribution change area is formed in a shape flattened in the second direction, and the discharge hole has a sectional shape flattened in the second direction.
According to such a manufacturing device for absorbent bodies, both the sectional shape of the flow path at the part on the aforementioned discharge hole side and the form of the aforementioned discharge hole are formed to have a shape flattened in the second direction. Therefore, the SAP distribution state that has been changed by the aforementioned distribution change area can be maintained in also the downstream side of the aforementioned distribution change area.

It is preferable that in the manufacturing device for an absorption body, the flow path of a part on the discharge hole side than the distribution change area is widened in the second direction as approaching the discharge hole.
According to such a manufacturing device for absorbent bodies, the flow path at the part on the aforementioned discharge hole side is widened in the aforementioned second direction so that the SAP distribution state changed by the aforementioned distribution change area can be maintained easier on the downstream side than the aforementioned distribution change area.

It is preferable that in the manufacturing device for an absorption body, a sectional shape of a flow path on an upstream side than the distribution change area is circular, an absorbent body made by accumulating the liquid absorbent fiber and the high-absorbent polymer to the accumulation portion is formed with an accumulation direction set as a thickness direction as well as having a longitudinal direction and a width direction in directions orthogonal to the thickness direction, and the second direction is parallel to the width direction of the absorbent body.
According to such a manufacturing device for absorbent bodies, the phenomenon of the amount of SAP increasing at the central portion in the second direction that may occur due to the circular sectional shape of the flow path on the aforementioned upstream side, is set off by the action of the diverted flow at the aforementioned distribution change area having a constricted part at the center in the second direction. Additionally, the corresponding second direction is parallel to the width direction of the absorbent body. Therefore, an approximately uniform SAP distribution can be realized in the aforementioned width direction.

It is preferable that in the manufacturing device for an absorption body, a pipe with a flow path having a perfect circular sectional shape is used for a part of the flow path that is on an upstream side of the distribution change area.
According to such a manufacturing device for absorbent bodies, a circular pipe being the most generally-used duct material can be applied to the portion on the upstream side of the polymer discharge tube so that a low cost manufacturing device can be realized.
Further, increasing the amount of SAP on both sides in the second direction as well as reducing the amount of SAP at the central portion in the second direction with the aforementioned distribution change area having a constricted part at the central portion in the second direction enables to solve the problem of the amount of SAP increasing at the central portion in the second direction which is likely to occur when the pipe is circular, and thereby SAP distribution in the second direction can be made uniform.

It is preferable that in the manufacturing device for an absorption body, a protrusion protruding from an inner wall face of the polymer discharge tube and being formed at the distribution change area allows to reduce a sectional area of a flow path of the distribution change area.
According to such a manufacturing device for absorbent bodies, the sectional area of the flow path can be reduced just by forming a protrusion in the distribution change area.

Further, a method of manufacturing an absorbent body according to the present invention includes accumulating liquid absorbent fiber in a first gas flowing through a duct to an accumulation portion, and discharging from a discharge hole of a polymer discharge tube being introduced into the duct a second gas having high-absorbent polymer mixed therein toward the accumulation portion, wherein a flow path of the second gas formed in the polymer discharge tube has at a location on the discharge hole side of the flow path a distribution change area where a distribution state of high-absorbent polymer is changed, and a sectional area of the flow path at the distribution change area is narrower than a sectional area of a flow path at a part adjacent on an upstream side as well as a downstream side of the distribution change area.
According to such a manufacturing method for absorbent bodies, operational advantages same as that of the aforementioned manufacturing device for absorbent bodies can be achieved.

### === Present Embodiment ===

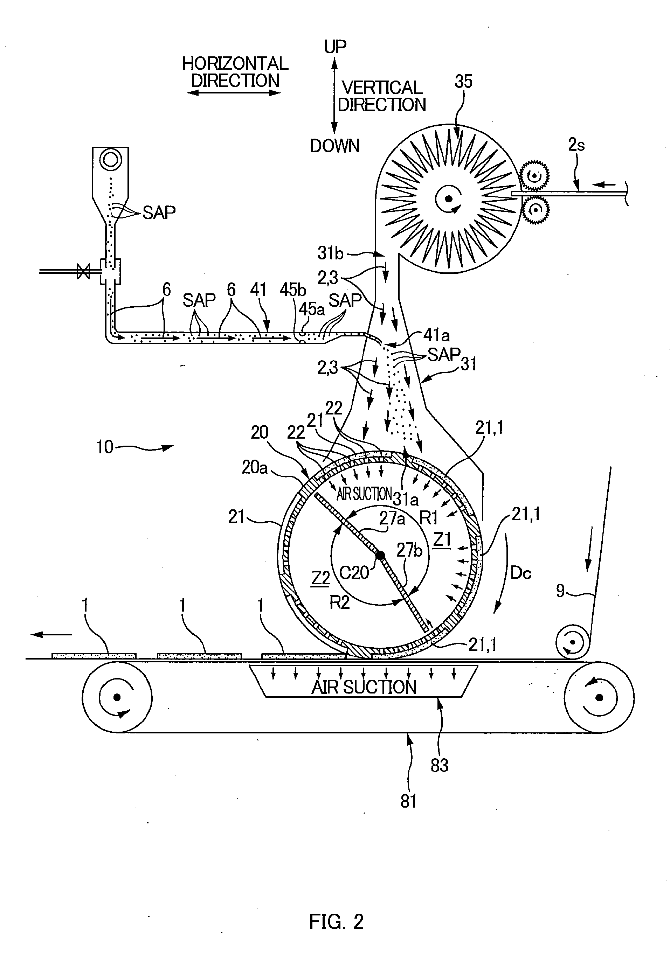

Fig. 2 is a schematic diagram of the manufacturing device 10 of absorbent body 1 according to the present embodiment showing a vertical sectional view of the manufacturing device 10 cut along the central portion thereof.

As shown in Fig. 2, the manufacturing device 10 for absorbent bodies 1 according to the present embodiment is a so-called fiber stacking device 10. In other words, this manufacturing device 10 includes a rotating drum 20 that has provided on the outer circumferential face 20a thereof a forming die 21 (corresponding to the accumulation portion) in a depressed form and that rotates in the circumferential direction Dc, a duct 31 that distributes pulp fiber 2 toward the outer circumferential face 20a of the rotating drum 20 to accumulate pulp fiber 2 in the aforementioned forming die 21 and form the absorbent body 1, and a belt conveyor 81 that is positioned on the downstream side of the circumferential direction Dc than the location at which the duct 31 is set, to convey the absorbent body 1 released from the forming die 21.

Note that in the description below, the circumferential direction Dc of the rotating drum 20 is also referred to as simply the "circumferential direction Dc" and the width direction of the rotating drum 20 (the direction perpendicular to the plane of the paper in Fig. 2) is also referred to as the "CD direction" or the "horizontal direction". Additionally, any direction within the plane intersecting this CD direction is also referred to as the "MD direction" and, for example, the aforementioned circumferential direction Dc is a part of the MD direction, the direction of the duct axis of duct 31 is a part of the MD direction and the duct axis of the later described polymer discharge tube 41 is also a part of the MD direction.

The rotating drum 20 has as its main body, a cylindrical body that rotates by being driven in the clockwise direction as one direction, about for example, a horizontal rotating shaft C20 in the CD direction. And on the circumferential face 20a thereof, multiple gas inlets 22 are formed at the bottom surface of each forming die 21 provided at a predetermined pitch in the circumferential direction Dc. Therefore, the pulp fibers 2 in the duct 31 flow along the flow 3 of air (corresponding to the first gas) created in the duct 31 by air intake through the aforementioned gas inlets 22, to be distributed and accumulated in the forming die 21. Thereby absorbent body 1 is formed in the forming die 21 with this direction of accumulation as the thickness direction thereof.

Note that, in the circumferential direction Dc, this air intake takes place in the first region R1 where the forming die 21 opposes the duct 31 but is stopped and does not take place at the second region R2 where the forming die 21 opposes the belt conveyor 81. Further, at the latter second region R2, the absorbent bodies 1 in the forming dies 21 are sequentially released from the forming dies 21 by air suction with the suction box 83 in the belt conveyor 81, and in this way the absorbent bodies 1 are transferred onto the belt conveyor 81 to be conveyed on the belt conveyor 81 thereafter. As an example of a configuration that performs air suction there can be given one that includes partition walls 27a, 27b that divide space at the inner circumferential side of the rotating drum 20 into zones in the circumferential direction Dc, and a blower, not shown, connected to zone Z1, between the plurality of zones Z1 and Z2, corresponding to the first region R1 at which air suction is to be performed for keeping negative pressure thereat. Note that it is a matter of course that the aforementioned air intake holes 22 of the rotating drum 20 and the aforementioned space on the inner circumferential side are in communication allowing air to flow.

Further, as shown in Fig. 2, sheet form members 9 such as non-woven fabric and tissue paper can be fed on this belt conveyor 81 for the absorbent bodies to be transferred thereon. And in this case, these sheet form members 9 become the surface sheet (the sheet that comes into contact with the wearer's skin) relating to disposable diapers and sanitary napkins.

As shown in Fig. 2, the duct 31 is for example a tubular member having an approximate rectangular section and is positioned above rotating drum 20 with the tube axis direction thereof oriented in the up-down direction (vertical direction) with regard to the MD direction while the distribution mouth 31a at the bottom end thereof covering over a predetermined area in the circumferential direction Dc above the outer circumferential face 20a of the rotating drum 20. Further, pulp fiber 2 made by pulverizing pulp sheet 2s by the pulverizer 35 is fed from the mouth 31b at the upper end being an end opposite the aforementioned distribution mouth 31a thereby creating inside the duct 31 an airflow 3 including pulp fibers 2 flowing from the upper side toward the lower side. Therefore, absorbent body 1 is formed by pulp fibers 2 being accumulated in the forming die 21 when the forming die 21 passes the location of the corresponding distribution mouth 31a along with the rotation of the rotating drum 20.

By the way, this duct 31 has inserted from the outer side thereof and positioned therein a polymer discharge tube 41 for injecting particulate SAP (high-absorbent polymer). And air 6 (corresponding to the second gas) having SAP mixed therein flows through this polymer discharge tube 41 along this airflow 6 for SAP to be discharged from the discharge hole 41a at the tip end of the polymer discharge tube 41 into the duct 31.

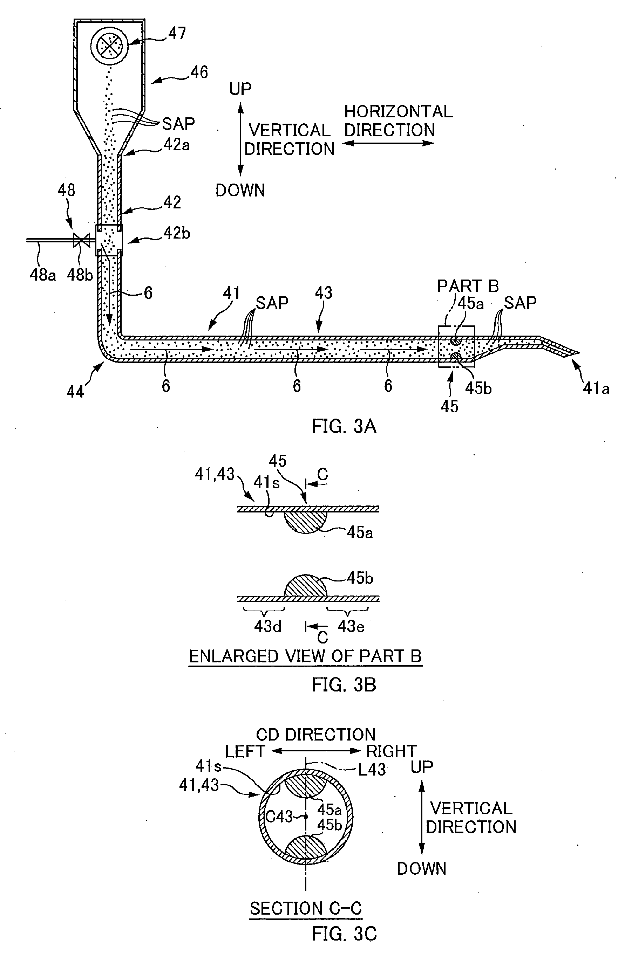

Fig. 3A shows a vertical sectional view of the polymer discharge tube 41 cut along the central portion thereof. Fig. 3B shows an enlarged view of part B in Fig. 3A, and Fig. 3C shows a sectional view taken along line C-C of Fig. 3B.

The polymer discharge tube 41 has a circular pipe (a cylindrical tube whose section is a perfect circle) bended in an L shape, for example, as its main body. Specifically, the polymer discharge tube 41 has a vertical duct portion 42 that has the duct axis oriented in the vertical direction to the MD direction and a horizontal duct portion 43 that has the duct axis oriented in the horizontal direction to the MD direction, and these two are connected by a bend duct 44. Further, the aforementioned discharge hole 41a is provided at the tip of the horizontal duct portion 43 whereas a SAP feed mechanism 46 for feeding SAP to the polymer discharge tube 41 is provided at the upper end 42a of the vertical duct portion 42. The SAP feed mechanism 46 has, for example, a screw feeder 47 at the upper portion thereof and SAP is volumetrically fed by allowing SAP to drop from the screw feeder 47 to the upper end 42a of the vertical duct portion 42. Further, a compressed air injection device 48 is connected at approximately the middle position 42b of this vertical duct portion 42. And compressed air of a predetermined pressure is permanently injected from this compressed air injection device 48 toward the aforementioned discharge hole 41 thereby creating an airflow 6 along the approximately tube axis direction at the portion in the tube on the downstream side of the aforementioned approximately middle position 42b, to allow SAP to flow along this airflow 6 to be discharged through the discharge hole 41a and into the duct 31.

As an example of a configuration of this compressed air injection device 48, there can be given one that includes a tank, not shown, that stores compressed air, a pipe 48a that connects this tank with the aforementioned vertical duct portion 42, a valve 48b that opens/closes the path of pipe 48a, and a compressor, not shown, that maintains within a predetermined range the pressure value of the compressed air in the aforementioned tank. And feeding of the compressed air into the polymer discharge tube 41 is controlled by controlling opening/closing of the valve 48b appropriately.

By the way, SAP is a particle whose median of its size is 300 to 500µm, for example, and its bulk density is for example 0.7 g/ml (700 kg/m³) being relatively heavy. Therefore, when SAP flow along the airflow 6 in the polymer discharge tube 41, they are not transferred in an evenly dispersed state within the section of the plow path of the polymer discharge tube 41 but rather in an uneven state. Thus when discharged into the duct 31 as it is, there is fear that the SAP distribution in the duct 31 will be uneven in the CD direction. Meanwhile, in many cases the forming die 21 of the drum 20 is commonly provided so that the absorbent body 1 to be formed is oriented in the CD direction, and in such a case, the accumulation distribution of SAP in the width direction of the absorbent body 1 would be uneven causing problems in fluid absorbing performance of the absorption article.

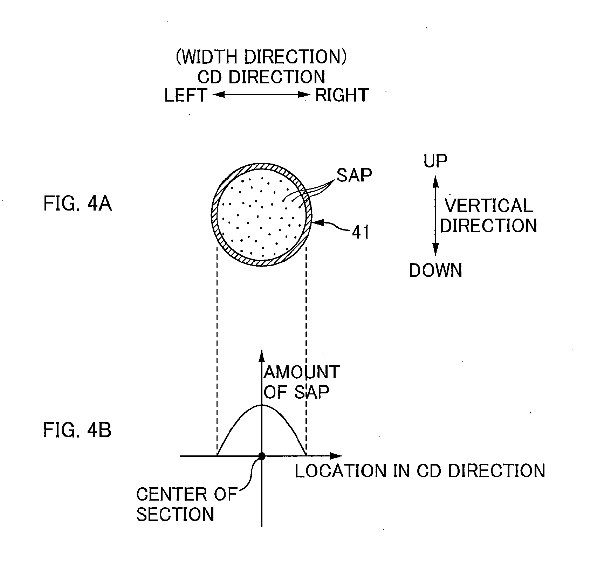

Even if SAP were to be evenly distributed within the section of the polymer discharge tube 41 as shown in Fig. 4A, the SAP distribution amount when seen in the CD direction is greater at the middle than the two sides in the CD direction due to the sectional shape of the polymer discharge tube 41 being a perfect circle. Therefore, when discharged as it is, the distribution would be such that the amount of SAP accumulated at the middle in the CD direction of the forming die 21 would be greater than that at the two sides. Being the case, equal distribution is prevented with SAP distribution in the absorbent body 1 being uneven in the width direction.

For this reason, a distribution change area 45 that changes the SAP distribution state is provided to the horizontal duct portion 43 of the polymer discharge tube 41 as shown in Figs. 3A through 3C. This distribution change area 45 is provided at a part on the discharge hole 41a side of the path of airflow 6 (the duct path between the aforementioned approximately middle position 42b being the location at which the compressed air injection device 48 is connected and the discharge hole 41a), that is to say, provided at a part closer to the discharge hole 41a than the aforementioned middle position 42b. And the sectional area of the flow path of this distribution change area 45 is narrower than the sectional area of the flow path of parts 43d, 43e adjacent at the upstream side and the downstream side of the distribution change area 45 as shown in Fig. 3B. And the SAP distribution state is changed with this.

For example, the horizontal duct portion 43 of the polymer discharge tube 41 in the examples shown in Figs. 3A to 3C is a circular pipe as described above, and the direction of flow along the flow path of the horizontal duct portion 43 is oriented in the horizontal direction with regard to the MD direction. Therefore, when the section with regard to the sectional area of this flow path is viewed as an imaginary plane with the direction of flow through the flow path as the normal direction, and two directions, orthogonal to each other as well as included in this imaginary plane, are regarded to be separated in the CD direction (corresponding to the second direction) and the vertical direction (corresponding to the first direction), since the sectional shape of the aforementioned flow path is a perfect circle, this sectional shape concerned is of a shape line symmetric with regard to the straight line L43 parallel to the aforementioned vertical direction running through the center C43 of the section.

And with regard to the sectional shape of the flow path at the distribution change area 45 in the example shown in Fig. 3C, the central portion in the CD direction is of a constricted shape with the clearance in the vertical direction narrower than the parts at the both sides (two end sides). Therewith, when SAP pass through this distribution change area 45, the SAP distribution state in the CD direction is changed such that the amount of SAP at the central portion in the CD direction is reduced and that at the parts at the both sides are increased.

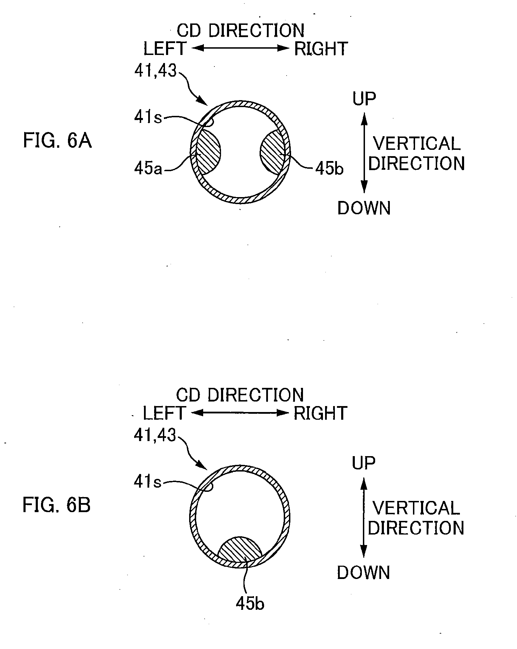

Various cases (such as those shown in Fig. 6A and Fig. 6B) according to the individual circumstances of the fiber stacking device 10 can be presumed as the above aspects for changing the state of distribution. Provided that the distribution change area 45 in concern is set in the polymer discharge tube 41 in all of the aspects for changing the distribution according to the present invention. Therefore, changes in SAP and airflow 6 in the polymer discharge tube 41 accompanied with the changes in the SAP distribution state can be effectively restrained from affecting the flow of pulp fiber 2 flowing on the outer side of the polymer discharge tube 41. That is, the SAP distribution state can be changed with the corresponding distribution change area 45 without having a large effect on the flow of pulp fiber 2 in the duct 31.

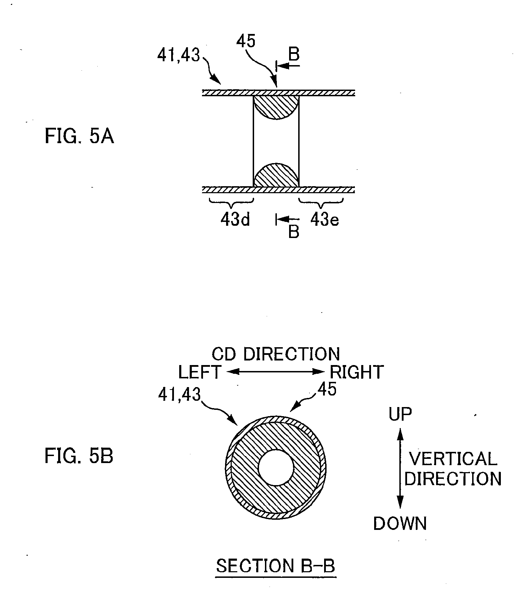

By the way, the aspect of changing the sectional area of the distribution change area 45 is not limited to the aspect where a part in the circumferential direction has a locally narrowed sectional shape as shown in Fig. 3C, but includes a case where the shape of the flow path, the shape of this section, is such that the diameter is evenly reduced along the whole circumference in the circumferential direction of the duct. And as shown in the vertical sectional view in Fig. 5A and the sectional view taken along line B-B of Fig. 5A shown in Fig. 5B are also included in the concept of the present invention. In other words, such aspects can change the SAP distribution state. For example in Fig. 5A, even if SAP were to flow in a distribution state with a predetermined unevenness along the flow path 43d at the upstream side than the distribution change area 45, SAP is forcefully collected to the center by the flow path with a reduced diameter when passing through this distribution change area 45. Therefore, when SAP after passing through this distribution change area 45 exits to flow path 43e where the diameter is widened once again, the SAP distribution state will have been changed to a more densely distributed state at the central portion of the section of the flow path.

Hereinafter, specific description on the structure of the distribution change area 45 will follow.

The distribution change area 45 in the example shown in Figs. 3B and 3C has a pair of hemispherical protrusions 45a, 45b formed to protrude inward in the radial direction from the inner wall face 41s of the polymer discharge tube 41. And one protrusion 45a is formed at the center in the CD direction of the ceiling plane, and another protrusion 45b is formed at the center in the CD direction of the bottom plane. In this way, the sectional shape of the flow path in the distribution change area 45 has a constricted shape whose clearance in the up-down direction (vertical direction) is widened at both sides than the central portion in the CD direction.

For such reason, when SAP flows along the airflow 6 through this distribution change area 45, the airflow 6 is divided into the left and right flows in the CD direction. And thereby, the SAP distribution state is changed to be sparse at the central portion and dense on the right and left sides thereof, compared to the SAP distribution state before passing through the distribution change area 45, and thereafter SAP is discharged through the discharge hole 41a into the duct 31.

Here in this example, since the sectional shape of the polymer discharge tube 41 is a perfect circle as described above, the amount of SAP distributed in the CD direction before passing through the distribution change area 45 is uneven such that the amount at the center in the CD direction is greater than that on its sides from the first place (see Fig. 4B) due to the sectional shape of the above-described perfect circular shape. For this reason, when passing this distribution change area 45 such unevenness is set off by the action of the diverted flow created by the sectional shape of the aforementioned distribution change area 45 and as a result, SAP is discharged into the duct 31 in a state with the amount thereof equalized in the CD direction. The equalized distribution state is basically maintained and as a result, the SAP accumulation distribution in the CD direction of the absorbent body 1 in the forming die 21 is also equalized. Accordingly, the SAP accumulation distribution in the width direction of the absorbent body 1 can be equalized by designing the position of the forming die 21 so that the CD direction becomes parallel to the width direction of the absorbent body 1.

Note that, in the example of Figs. 3B and 3C, protrusions 45a, 45b were provided to the ceiling plane and the bottom plane, respectively, in a manner opposing each other to equalize the accumulation distribution of SAP in the CD direction. However, the location of the protrusions 45a, 45b and their numbers can be appropriately determined according to the individual circumstances of each fiber stacking device 10.

As shown in the sectional diagram in Fig. 6A, for example, the protrusions 45a, 45b can be respectively provided on the pair of right and left side faces (the set of faces arranged in the CD direction) of the inner wall face 41s of the polymer discharge tube 41. In this way, the SAP distribution after passing the distribution change area 45 will be changed to a state where the distribution is dense at the central portion in the CD direction and sparse at the pair of right and left side faces compared to that before passing the distribution change area 45. Note that when the SAP distribution is made uneven intentionally in the CD direction, the protrusions 45a, 45b can be provided to only one side face of the two side faces.

Alternatively, the protrusion 45b can be provided to only one of the ceiling face and the bottom face. And in this case where only one protrusion is provided, the protrusion may be provided on the bottom face as show in Fig. 6B. This is because the SAP distribution in the vertical direction is thought to be uneven such that the amount of SAP in the lower half of the space is greater than that in the upper half of the space. Therefore it is understood that the protrusion 45b provided at the bottom face would be more effective in contributing to separate the SAP flow into the right and left flows in the CD direction.

Taking into account the number of the aforementioned protrusions 45a, 45b set, the detail conditions such as the height, dimension and shape of the protrusions 45a, 45b are drawn from actual SAP distribution measuring tests and the like conducted with the inner diameter of the polymer discharge tube 41, SAP discharge amount per unit time, amount of airflow per unit time, shape of the discharge hole 41a and the like as parameters.

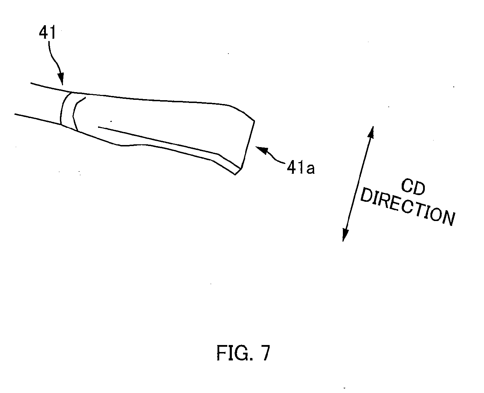

By the way, in the examples shown in Figs. 3A to 3C, it is preferable that the shape of the flow path from this distribution change area 45 to the discharge hole 41a on the downstream side thereof is a shape that is capable of maintaining even SAP distribution in the CD direction. Fig. 7 is an example thereof showing a perspective view of a portion proximate the discharge hole 41a of the polymer discharge tube 41 and the flow paths in the examples shown in this Fig. 7 and Fig. 3A have a flattened sectional shape. To be more specific, the flow path is formed such that the dimensions in the up-down direction (vertical direction) is reduced stepwise or continuously toward the discharge hole 41a or the dimension in the CD direction is increased stepwise or continuously toward the discharge hole 41a thereby forming the flow path to have an approximately rectangular sectional shape flattened with the dimension in the vertical direction of the flow path being smaller than that in the CD direction. And at the tip thereof, the aforementioned discharge hole 41a is provided. Note that this discharge hole 41a also has a sectional shape flattened in the CD direction, to be specific, the shape of the mouth portion is approximately rectangular with the CD direction as its longitudinal direction.

Here, it is preferable that the dimension of the discharge hole 41a in the CD direction is equal to or larger than the inner diameter of the polymer discharge tube 41 and equal to or smaller than the dimension of the absorbent body 1 in the CD direction. Further, it is preferable that the dimension of the discharge hole 41a in the up-down direction is such that the area of the discharge hole 41a is equal to or smaller than the sectional area of the polymer discharge tube 41.

Such shapes of the flow paths with a flattened sectional shape is formed by, for example, collapsing the portion proximate the discharge hole 41 a of the polymer discharge tube 41 in the up-down direction while widening in the CD direction to have a flattened tubular shape (refer to Figs. 7 and 3A).

### === Other Embodiments ===

Hereinabove, explanation on the embodiments of the present invention have been given, however, the present invention is not limited to such embodiments and modifications such as those in the following can be made.

In the aforementioned embodiment, as in Figs. 3B and 3C, protrusions having hemispherical shapes have been exemplified as the protrusions 45a, 45b provided to the distribution change area 45. However, the shape of the protrusions is not limited to such. For example, the shape can be pyramidal or conical such as a triangular pyramid or a circular cone or a unique shape having a plurality of different curved surfaces and planes combined. Note that, when a protrusion in a pyramidal or conical shape is provided, the bottom side of the protrusion can be positioned to face the inner wall face 41s of the polymer discharge tube 41.

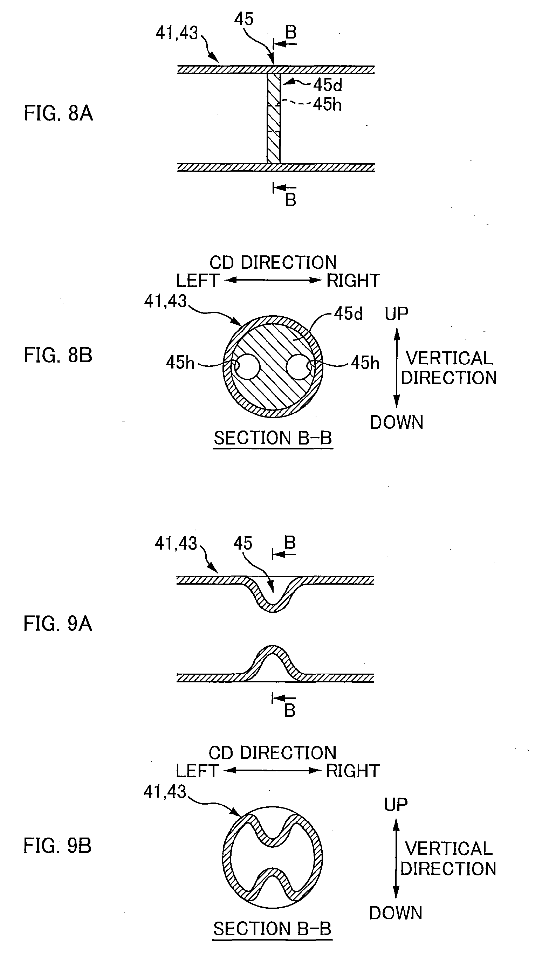

Further, as a structure for making the sectional area of the flow path of the distribution change area 45 smaller than the sectional area of the portions 43d, 43e adjacent the upstream side and downstream side thereof is not limited to protrusions 45a, 45b. For example, as shown in the vertical sectional view in Fig. 8A and the sectional view taken along line B-B of Fig. 8A in Fig. 8B, a configuration having a baffle plate 45d formed with through holes 45h, 45h in the thickness direction set to the flow path in the distribution change area 45 may be used. Moreover, as shown in the vertical sectional view in Fig. 9A and the sectional view taken along line B-B of Fig. 9A in Fig. 9B, a part of the polymer discharge tube 41 may be collapsed to narrow the flow path and be set as the distribution change area 45.

In the aforementioned embodiment, the polymer discharge tube 41 was configured with a circular pipe having a perfect circular sectional shape. However, the configuration is not limited to such and any configuration can be applied as long as a tubular material is used. For example, a pipe whose section is a polygonal shape such as a square pipe or a circular pipe whose section is such as an ellipsoidal shape can be used.

In the aforementioned embodiment, forming die 21 formed on the outer circumferential face 20a of the rotating drum, and formed to have a depressed shape was shown as an example of the accumulation portion, however, the accumulation portion is not limited to such. For example, the outer circumferential face 20a configured to have a generally smooth surface with suction force acting only in this predetermined area of this outer circumferential area 20a can be used to form absorbent bodies 1 by accumulating pulp fibers 2 and SAP at this predetermined area as the aforementioned accumulation portion. Further, a chain conveyor or a belt conveyor and the like can be used instead of the rotating drum 20. In other words, the forming die 21 can be made to travel in a predetermined circumferential orbit with a corresponding conveyor while the aforementioned duct 31 is positioned at a predetermined location along the circumferential orbit.

In the aforementioned embodiment, pulp fiber 2 (pulp pulverized into fibrous form) was exemplified as liquid absorbent fiber, however, various material used for the absorbent body 1 of absorbent articles such as conventional sanitary napkins, disposable diapers and the like can be used as this liquid absorbent fiber without special requirements. For example, cellulosic short fiber such as rayon fiber and cotton fiber or synthetic short fiber such as polythene fiber and the like can be used. These fibers can be used alone or, two types or more of them combined.

In the aforementioned embodiment, specific examples of high-absorbent polymer (SAP) was not described, however, various material used for the absorbent body 1 of absorbent articles such as conventional sanitary napkins, disposable diapers and the like can be used as this SAP without special requirements. For example, starch material, cellulosic material, synthetic polymer material and the like can be used. Here, SAP is generally in a particle form. As SAP, it is preferable that it is liquid absorbing and retentive of 20 times or more of its own weight and having a characteristic of gelation as well. For example, starch-acrylic acid (sodium) grafted copolymer, saponifiable material of starch-acrylonitrile copolymer, cross-linked sodium carboxymethylcellulose, acrylic acid (sodium) copolymer and the like are preferable. The above SAP can be used alone or, two types or more of them combined.

In the aforementioned embodiment, air 3, 6 were exemplified as examples of the first gas and the second gas, however, the gases are not limited to such. That is, as long as gas can be a medium for transferring liquid absorbing fiber and SAP, and does not cause chemical reaction with these liquid absorbing fiber and SAP, the gas need not be air and for example, nitrogen can be used.

In the aforementioned embodiment, the duct 31 continuous in the vertical direction was positioned above the rotating drum 20, and its distribution mouth 31a made to cover the outer circumferential surface of the rotating drum 20 from above so that the flow path of the airflow 3 including pulp fiber 2 was formed to extend in the vertical direction, however, the positioning is not limited to such. For example, a duct 31 continuous in a horizontal or a diagonal direction to the MD direction can be positioned with the flow path of the airflow 3 formed in the horizontal or diagonal direction.

In the aforementioned embodiment, only one distribution change area 45 was set in the tube axis direction of the polymer discharge tube 41, however, it's number is not limited to one and can be set at a plurality of locations along the tube axis direction.

### [Reference Signs List]

1 absorbent body, 2 pulp fiber (liquid absorbent fiber),
2s pulp sheet, 3 airflow (first gas, air),
6 airflow (second gas, air), 9 sheet form member,
10 fiber stacking device (manufacturing device),
20 rotating drum, 20a outer circumferential face,
21 forming die (accumulation portion), 22 air intake holes,
27a partitioning wall, 27b partitioning wall, 31 duct,
31a distribution mouth, 31b mouth at upper end,
35 pulverizer, 41 polymer discharge tube, 41a discharge hole,
41s inner wall face, 42 vertical duct portion, 42a upper end,
42b approximately middle position, 43 horizontal duct portion,
43d part adjacent to the upstream side of the distribution change area,
43e part adjacent to the downstream side of the distribution change area,
44 bend duct, 45 distribution change area, 45a protrusion,
45b protrusion, 45d baffle plate, 45h through hole,
46 SAP feed mechanism, 47 screw feeder,
48 compressed air injection device, 48a pipe, 48b valve,
81 belt conveyor, 83 suction box, SAP high-absorbent polymer,
R1 first region, R2 second region, Z1 zone, Z2 zone,
C20 rotating shaft, C43 center of section

## Claims

1. A manufacturing device for an absorption body comprising:
an accumulation portion that is to have accumulated thereto liquid absorbent fiber in a first gas flowing through a duct; and
a polymer discharge tube that is introduced into the duct and that discharges from a discharge hole a second gas having high-absorbent polymer mixed therein toward the accumulation portion;
wherein a flow path of the second gas formed in the polymer discharge tube has at a location on the discharge hole side of the flow path a distribution change area where a distribution state of high-absorbent polymer is changed, and
a sectional area of the flow path at the distribution change area is narrower than a sectional area of a flow path at a part adjacent on an upstream side as well as a downstream side of the distribution change area.

2. The manufacturing device for an absorption body according to claim 1, wherein
a section relating to a sectional area of the flow path is an imaginary plane having a direction of flow through the flow path in a normal direction thereof,
a shape of the section, when two directions orthogonal to each other and included in the imaginary plane are set as a first direction and a second direction, respectively, is a shape line-symmetric against a straight line parallel to the first direction running through a center of the sectional area, and
a sectional shape of a flow path at the distribution change area being such that a clearance in the first direction at the central portion in the second direction is of a constricted shape narrower than parts on two sides of the distribution change area.

3. The manufacturing device for an absorption body according to claim 2, wherein
a sectional shape of a flow path of a part on the discharge hole side than the distribution change area is formed in a shape flattened in the second direction, and
the discharge hole has a sectional shape flattened in the second direction.

4. The manufacturing device for an absorption body according to claim 2 or 3, wherein
the flow path of a part on the discharge hole side than the distribution change area is widened in the second direction as approaching the discharge hole.

5. The manufacturing device for an absorption body according to one of claims 2 to 4, wherein
a sectional shape of a flow path on an upstream side than the distribution change area is circular,
an absorbent body made by accumulating the liquid absorbent fiber and the high-absorbent polymer to the accumulation portion is formed with an accumulation direction set as a thickness direction as well as having a longitudinal direction and a width direction in directions orthogonal to the thickness direction, and
the second direction is parallel to the width direction of the absorbent body.

6. The manufacturing device for an absorption body according to one of claims 2 to 5, wherein
a pipe with a flow path having a perfect circular sectional shape is used for a part of the flow path that is on an upstream side of the distribution change area.

7. The manufacturing device for an absorption body according to one of claims 1 to 6, wherein
a protrusion protruding from an inner wall face of the polymer discharge tube and being formed at the distribution change area allows to reduce a sectional area of a flow path of the distribution change area.

8. A method of manufacturing an absorbent body comprising:
accumulating liquid absorbent fiber in a first gas flowing through a duct to an accumulation portion; and
discharging from a discharge hole of a polymer discharge tube being introduced into the duct a second gas having high-absorbent polymer mixed therein toward the accumulation portion;
wherein a flow path of the second gas formed in the polymer discharge tube has at a location on the discharge hole side of the flow path a distribution change area where a distribution state of high-absorbent polymer is changed, and
a sectional area of the flow path at the distribution change area is narrower than a sectional area of a flow path at a part adjacent on an upstream side as well as a downstream side of the distribution change area.
